# EUROPÄISCHE PATENTSCHRIFT

(11) **EP 1 912 990 B1**
(45) Veröffentlichungstag und Bekanntmachung des Hinweises auf die Patenterteilung: **26.06.2013**
(21) Anmeldenummer: 06777973.6
(22) Anmeldetag: 26.07.2006
(51) Int. Cl.: C07D 473/04, A61K 31/522, A61P 3/10

(54) **HYDROCHLORIDE UND HYDRATE VON 1-[(3-CYANO-PYRIDIN-2-YL) METHYL]-3-METHYL-7-(2-BUTIN-1-YL)-8-(3-AMINO-PIPERIDIN-1-YL)-XANTHIN, DEREN HERSTELLUNG UND DEREN VERWENDUNG ALS ARZNEIMITTEL**
HYDROCHLORIDES AND HYDRATES OF 1-[(3-CYANOPYRIDIN-2-YL)METHYL]-3-METHYL-7-(2-BUTYN-1-YL)-8-(3-AMINOPIPERIDIN-1-YL)XANTHINE, THEIR PREPARATION AND THEIR USE AS MEDICAMENTS
HYDROCHLORURES ET HYDRATES DE 1-[(3-CYANO-PYRIDIN-2-YL) METHYL]-3-METHYL-7-(2-BUTIN-1-YL)-8-(3-AMINO-PIPERIDIN-1-YL)-XANTHINE, LEUR PRODUCTION, ET LEUR UTILISATION EN TANT QUE MÉDICAMENTS

(30) Priorität: 30.07.2005 DE 102005035891
(43) Veröffentlichungstag der Anmeldung: 23.04.2008
(60) Teilanmeldung: 12182384.3
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: PFRENGLE, Waldemar, 88400 Biberach (DE); SIEGER, Peter, 88441 Mittelbiberach (DE)
(74) Vertreter: Simon, Elke Anna Maria
(86) Internationale Anmeldenummer: PCT/EP2006/064657
(87) Internationale Veröffentlichungsnummer: WO 2007/014886

(56) Entgegenhaltungen:
- EP-A2- 1 338 595
- WO-A-02/02560
- WO-A-02/068420
- WO-A-2005/085246
- WO-A-2006/048427
- WO-A2-2004/018468

## Beschreibung

Gegenstand der vorliegenden Erfindung sind Hydrochloridsalze der Formel 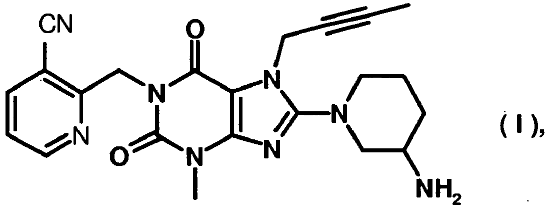 , welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die ein Hydrochloridsalz der Verbindung der allgemeinen Formel (I) enthaltenden Arzneimittel sowie Verfahren zu deren Herstellung.

Xanthinderivate mit einer hemmenden Wirkung auf DPP-IV sind bereits aus WO 02/068420, WO 02/02560, WO 03/004496, WO 03/024965, WO 04/018468, WO 04/048379, JP 2003300977 und EP 1 338 595 bekannt.

Aufgabe der Erfindung ist es, neue Salze mit vorteilhaften Eigenschaften für die pharmazeutische Anwendung bereitzustellen.

Neben der eigentlichen Wirksamkeit für die gewünschte Indikation muß ein Wirkstoff noch weiteren Anforderungen gerecht werden, um als Arzneimittel zum Einsatz gelangen zu können. Diese Parameter sind zu einem großen Teil mit der physikochemischen Beschaffenheit des Wirkstoffs verbunden.

Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung der Arzneimittelzusammensetzungen verwendete Arzneiwirkstoff sollte daher eine hohe Stabilität aufweisen, die auch unter verschiedenen Umgebungsbedingungen gewährleistet sein muß. Dies ist zwingend erforderlich, um zu verhindern, dass Arzneimittelzusammensetzungen Verwendung finden, in denen neben tatsächlichem Wirkstoff beispielsweise Abbauprodukte desselben enthalten sind. In einem solchen Fall könnte ein in pharmazeutischen Formulierungen vorgefundener Gehalt an Wirkstoff niedriger sein als spezifiziert.

Die Absorption von Feuchtigkeit vermindert den Gehalt an Arzneiwirkstoff wegen der durch die Wasseraufnahme verursachten Gewichtszunahme. Zur Aufnahme von Feuchtigkeit neigende Arzneimittel müssen während der Lagerung vor Feuchtigkeit geschützt werden, beispielsweise durch Zusatz von geeigneten Trockenmitteln oder durch Lagerung des Arzneimittels in einer vor Feuchtigkeit geschützten Umgebung. Zudem kann die Aufnahme von Feuchtigkeit den Gehalt an Arzneiwirkstoff während der Herstellung vermindern, wenn das Arzneimittel der Umgebung ohne jeglichen Schutz vor Feuchtigkeit ausgesetzt wird. Vorzugsweise sollte ein Arzneimittelwirkstoff daher nur in geringem Maße hygroskopisch sein.

Da die Kristallmodifikation eines Wirkstoffs für den reproduzierbaren Wirkstoffgehalt einer Darreichungsform von Bedeutung ist, besteht die Notwendigkeit, eventuell existierenden Polymorphismus eines kristallin vorliegenden Wirkstoffs bestmöglich aufzuklären. Sofern verschiedene polymorphe Modifikationen eines Wirkstoffs auftreten, sollte gewährleistet sein, dass sich die kristalline Modifikation der Substanz in der späteren Arzneimittelzubereitung nicht verändert. Andernfalls könnte dies die reproduzierbare Wirksamkeit des Medikaments nachteilig beeinflussen. Bevorzugt sind vor diesem Hintergrund Wirkstoffe, die nur durch geringen Polymorphismus gekennzeichnet sind.

Ein weiteres Kriterium, welches je nach Wahl der Formulierung oder nach Wahl des Herstellungsverfahrens der Formulierung von unter Umständen herausragender Bedeutung ist, ist die Löslichkeit des Wirkstoffs. Werden beispielsweise Arzneimittellösungen (etwa für Infusionen) bereitgestellt, so ist eine ausreichende Löslichkeit des Wirkstoffs in physiologisch verträglichen Lösemitteln unverzichtbar. Auch für oral zu applizierende Arzneimittel ist eine ausreichende Löslichkeit des Wirkstoffs von großer Wichtigkeit.

Der vorliegenden Erfindung liegt die Aufgabe zugrunde, einen Arzneimittelwirkstoff bereitzustellen, der nicht nur durch eine hohe pharmakologische Wirksamkeit gekennzeichnet ist, sondern ferner den vorstehend genannten physikochemischen Anforderungen bestmöglich gerecht wird.

Überraschenderweise wurde nun gefunden, dass die Salze der Verbindung der Formel I mit Salzsäure, und deren Enantiomere diese Aufgabe erfüllen. Besonders geeignet im Sinne dieser Erfindung sind das Mono- und das Dihydrochlorid sowie deren Enantiomere.

Folgende Bezeichnungen werden synonym verwendet:
Salz mit Salzsäure - Hydrochlorid

Gegenstand der Erfindung sind daher die Salze von 1-[(3-Cyano-pyridin-2-yl)methyl]-3-methyL-7-(2-butin-1-yL)-8-(3-amino-piperidin-1-yL)-xanthin mit Salzsäure, sowie deren Enantiomere. Dazu zählen das Mono- und das Dihydrochlorid von 1-[(3-Cyano-pyridin-2-yl)methyl]-3-methyl-7-(2-butin-1-yl)-8-[(R)-3-amino-piperidin-1-yl]-xanthin und von 1-[(3-Cyano-pyridin-2-yl)methyl]-3-methyl-7-(2-butin-1-yl)-8-[(S)-3-amino-piperidin-1-yl]-xanthin sowie ihrer Gemische einschließlich des Racemats. Ein weiterer Erfindungsgegenstand sind pharmazeutische Zusammensetzungen enthaltend mindestens eines der oben genannten Salze sowie Verfahren zur Herstellung von Arzneimitteln.

Im Hinblick auf die Fähigkeit, die DPP-IV Aktivität zu hemmen, sind die erfindungsgemäßen Salze der allgemeinen Formel I geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ 1 und Typ 2, Prädiabetes, Verminderung der Glukosetoleranz oder Veränderungen im Nüchternblutzucker, diabetische Komplikationen (wie z.B. Retinopathie, Nephropathie oder Neuropathien), metabolische Azidose oder Ketose, reaktiver Hypoglykämie, Insulinresistenz, Metabolischem Syndrom, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Weiterhin sind die erfindungsgemäßen Verbindungen zur Behandlung entzündlicher Erkrankungen der Atemwege einsetzbar. Ebenso sind sie zur Prävention und Therapie von chronischen entzündlichen Darmerkrankungen wie z.B. Reizdarmsyndrom (IBS), Morbus Crohn oder Colitis ulcerosa ebenso wie bei Pankreatitis geeignet. Des weiteren wird erwartet, daß sie bei jeglicher Art von Verletzung oder Beeinträchtigung im Gastrointestinaltrakt eingesetzt werden können wie auch z.B. bei Kolitiden und Enteriden. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Auf der anderen Seite sind diese Substanzen geeignet, die Motilität der Spermien zu beeinflussen und sind damit als Kontrazeptiva zur Verwendung beim Mann einsetzbar. Des weiteren sind die Substanzen geeignet, Mangelzustände von Wachstumshormon, die mit Minderwuchs einhergehen, zu beeinflussen, sowie bei allen Indikationen sinnvoll eingesetzt werden können, bei denen Wachstumshormon verwendet werden kann. Die erfindungsgemäßen Verbindungen sind auf Grund ihrer Hemmwirkung gegen DPP IV auch geeignet zur Behandlung von verschiedenen Autoimmunerkrankungen wie z.B. rheumatoide Arthritis, Multiple Sklerose, Thyreoditiden und Basedow'scher Krankheit etc.. Darüberhinaus können sie eingesetzt werden bei viralen Erkrankungen wie auch z.B. bei HIV Infektionen, zur Stimulation der Blutbildung, bei benigner Prostatahyperplasie, bei Gingivitiden, sowie zur Behandlung von neuronalen Defekten und neur-degenerativen Erkrankungen wie z.B. Morbus Alzheimer. Beschriebene Verbindungen sind ebenso zu verwenden zur Therapie von Tumoren, insbesondere zur Veränderung der Tumorinvasion wie auch Metastatisierung, Beispiele hier sind die Anwendung bei T-Zell Lymphomen, akuter lymphoblastischer Leukämie, zellbasierende Schilddrüsenkarzinome, Basalzellkarzinome oder Brustkarzinome. Weitere Indikationen sind Schlaganfall, Ischämien verschiedenster Genese, Morbus Parkinson und Migräne. Darüberhinaus sind weitere Indikationsgebiete follikuläre und epidermale Hyperkeratosen, erhöhte Keratinozytenproliferation, Psoriasis, Enzephalomyelitiden, Glomerulonephritiden, Lipodystrophien, sowie psychosomatische, depressive und neuropsychiatrische Erkrankungen verschiedenster Genese.

Die erfindungsgemäßen Salze können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. GI 262570) und -Antagonisten, PPAR-gamma/alpha Modulatoren (z.B. KRP 297), PPAR-gamma/alpha/delta Modulatoren, AMPK-Aktivatoren, ACC1 und ACC2 Inhibitoren, DGAT-Inhibitoren, SMT3-Rezeptor-Agonisten, 11β-HSD-Inhibitoren, FGF19-Agonisten oder -Mimetika, alpha-Glucosidasehemmer (z.B. Acarbose, Voglibose), andere DPPIV Inhibitoren, alpha2-Antagonisten, Insulin und Insulinanaloga, GLP-1 und GLP-1 Analoga (z.B. Exendin-4) oder Amylin. Daneben sind Kombinationen mit SGLT2-Inhibitoren wie T-1095 oder KGT-1251 (869682), Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phospho enolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha Agonisten, PPAR-delta Agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder LXRalpha Antagonisten, LXRbeta Agonisten oder LXRalpha/beta Regulatoren oder Wirkstoffe zur Behand lung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder ß₃-Agonisten wie SB-418790 oder AD-9677 ebenso wie Agonisten des 5HT2c Rezeptors möglich.

Daneben ist eine Kombination mit Medikamenten zur Beeinflussung des Bluthochdrucks wie z.B. All Antagonisten oder ACE Inhibitoren, Diuretika, β-Blocker, Ca-Antagonisten und anderen oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

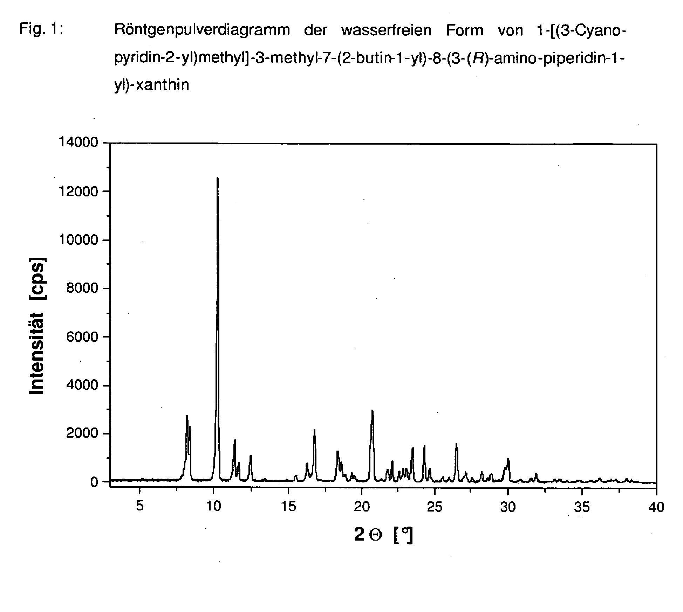

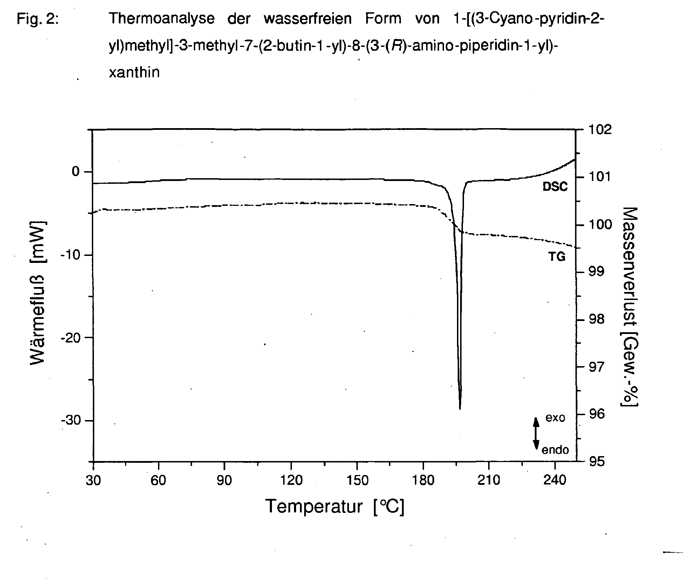

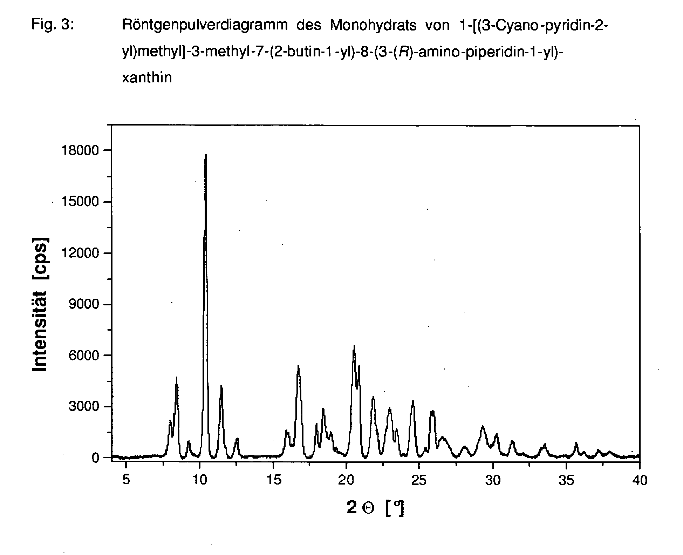

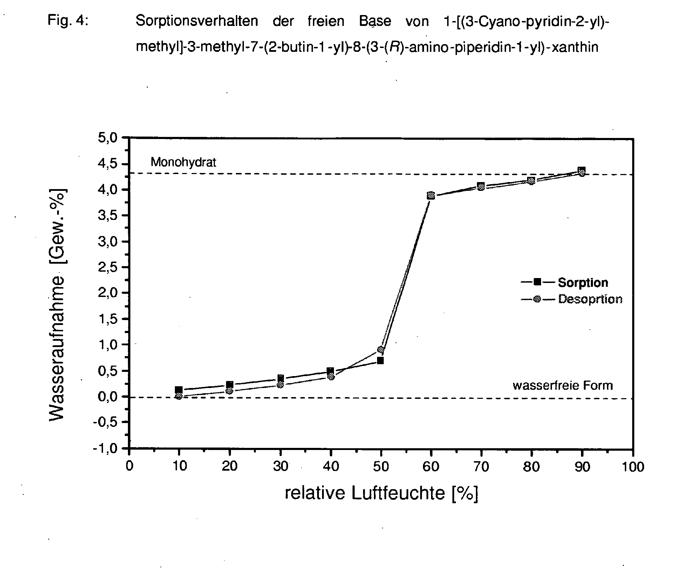

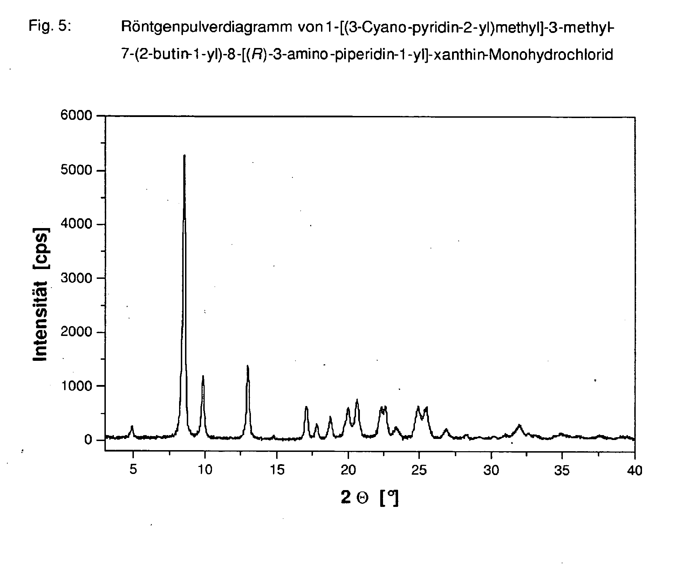

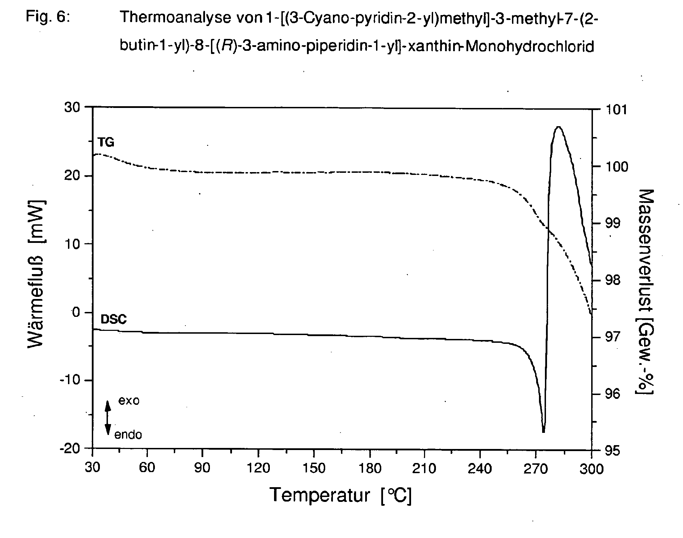

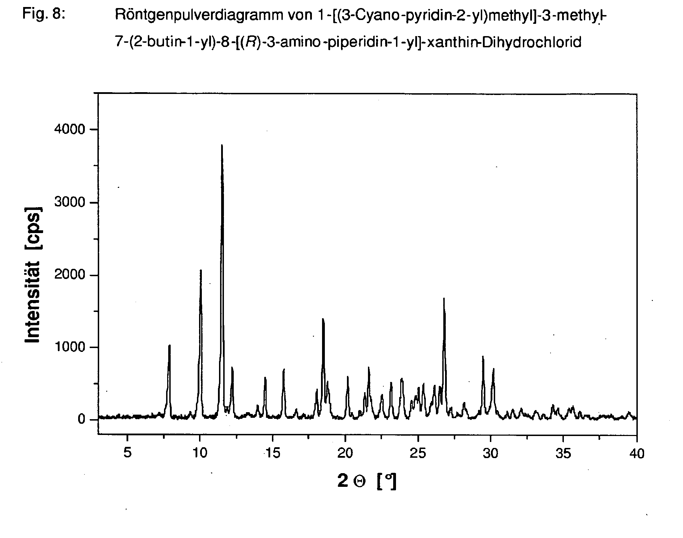

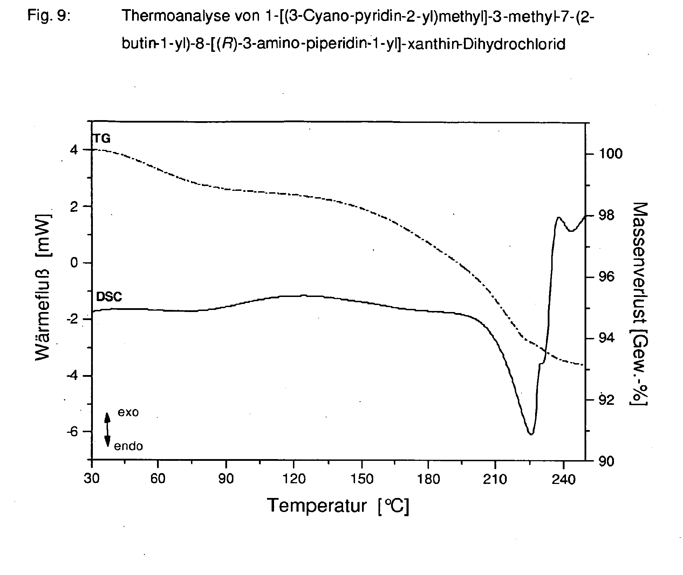

Die Figuren 1, 3, 5 und 8 zeigen die Röntgenpulverdiagramme der Beispiele 2 (wasserfreie Form sowie Monohydrat), 3 und 4; die Figuren 2, 6 und 9 geben die Thermoanalysen der Beispiele 2, 3 bzw. 4 wieder und die Figuren 4, 7 und 10 zeigen das Sorptionsverhalten der freien Base sowie des Mono- und Dihydrochlorides von 1-[(3-Cyano-pyridin-2-yl)methyl]-3-methyl-7-(2-butin-1-yl)-8-(3-(R)-amino-piperidin-1-yl)-xanthin (Beispiel 2, 3 und 4).

Die nachfolgenden Beispiele solle n die Erfindung näher erläutern. Beispiel 2 ist ein Vergleichsbeispiel.

### Beispiel 1

### D-Weinsäuresalz des R-Enantiomers von 3-(Phthalimido)piperidin

### a. Hydrierung:

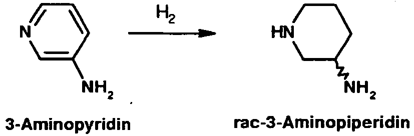

10,00 kg (106,25 mol) 3-Aminopyridin, 500 g Aktivkohle techn. und 65 Liter Essigsäure werden im Hydrierreaktor vorgelegt. 50 g Nishimura Katalysator (ein kommerziell erhältlicher Rhodium/Platin Mischkatalysator) werden in 2,5 Liter Essigsäure aufgeschlemmt zugeben und mit 2,5 Liter Essigsäure nachgespült. Es wird bei 50°C und 100 bar Wasserstoffüberdruck bis zum Stillstand der Wasserstoffaufnahme hydriert und anschließend 30 Minuten bei 50°C nachhydriert. Der Katalysator und die Aktivkohle werden abfiltriert und mit 10 Liter Essigsäure nachwaschen.

Die Reaktion gelingt auch unter weniger drastischen Drücken.

### b. Acylierung:

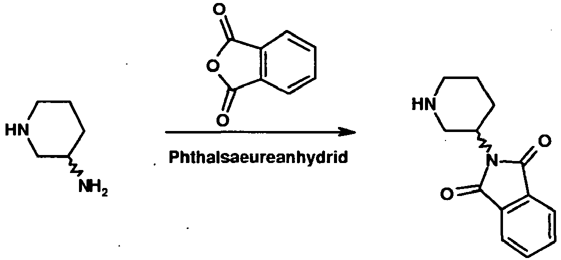

15,74 kg (106,25 mol) Phthalsäureanhydrid werden im Reaktor vorgelegt und mit dem Filtrat aus der Hydrierung versetzt. Es wird mit 7,5 Liter Essigsäure nachgespült, und anschließend wird die Reaktionsmischung zum Rückfluß erhitzt, wobei innerhalb einer Stunde ca, 30% der eingesetzten Essigsäure abdestilliert werden. Die Reaktionslösung wird auf 90°C abgekühlt.

### c. Racematspaltung:

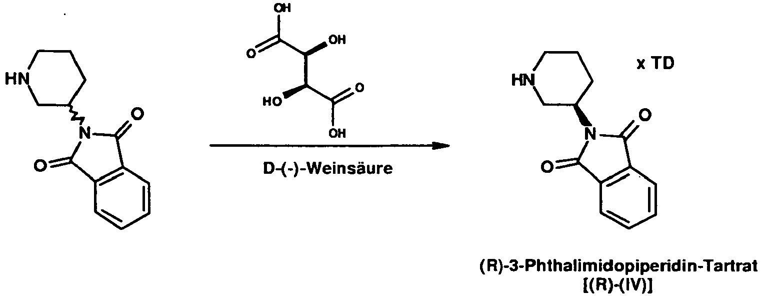

Eine auf 50°C erwärmte Lösung von 11,16 kg D-(-)-Weinsäure (74,38 mol) in 50 Liter absolutem Ethanol wird bei 90°C zur Acylierungsreaktionslösung zudosiert. Es wird mit 10 Liter Ethanol absolut nachgespült und 30 Minuten bei 90°C nachgerührt, wobei das Produkt kristallisiert. Nach dem Abkühlen auf 5°C wird das Produkt abzentrifugiert und mit Ethanol absolut gewaschen.

### d. Umkristallisation:

Das feuchte Rohprodukt wird in einer Mischung von 50 Liter Aceton und 90 Liter Wasser so lange zum Rückfluß erhitzt, bis eine Lösung entstanden ist. Anschliessend wird auf 5°C abgekühlt, wobei das Produkt auskristallisiert. Die Suspension wird bei 5°C 30 Minuten nachgerührt, das Produkt wird abzentrifugiert und zuletzt mit einer Mischung aus 20 Liter Aceton und 10 Liter Wasser gewaschen. Es wird im Trockenschrank unter Inertisierung bei 45°C getrocknet.
Ausbeuten: 11,7-12,5 kg

### Beispiel 2

### Herstellung von 1-[(3-Cyano-pyridin-2-yl)methyl]-3-methyl-7-(2-butin-1-y)-8-[(R)-3-amino-piperidin-1-yl]-xanthin-Base

### a. 3-Cyano-2-(chlormethyl)-pyridin

165,5 g (0,98 mol) 2-Hydroxymethyl-3-pyridincarboxamid werden zusammen mit 270 ml Phosphoroxychlorid für 1 Stunde auf 90-100°C erhitzt. Das Reaktionsgemisch wird auf Raumtemperatur abgekühlt und anschließend in ca. 800 ml 50-60°C warmes Wasser eingetropft. Nach Hydrolyse des Phosphoroxychlorids wird unter Kühlung mit Natronlauge neutralisiert, wobei das Produkt ausfällt. Es wird abfiltriert, mit 300 ml Wasser gewaschen und anschließend bei 35-40°C getrocknet.
Ausbeute: 122,6 g (82% d. Th.)

### b. 1-[(3-Cyano-pyridin-2-yl)methyl]-3-methyl-7-(2-butin-1-yl)-8-brom-xanthin

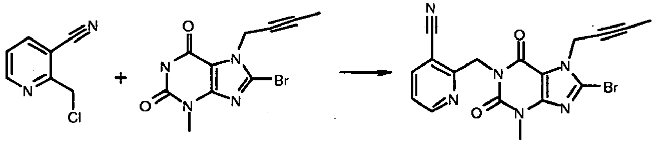

202 g (0,68 mol) 3-Methyl-7-(2-butin-1-yl)-8-brom-xanthin , 188,5 g (1,36 mol) Kalium carbonat wasserfrei und 1 ,68 Liter N-Methyl-2-pyrrolidon werden im Reaktor vorgelegt und auf 70°C erwärmt. Anschließend werden 119 g (0,75 mol) 2-Chlormethyl-3-cyano-pyridin in 240 ml N-Methyl-2-pyrrolidin (NMP) zugetropft. Der Reaktorinhalt wird für 19 Stunden bei 70°C gerührt. Nach beendeter Reaktion wird der Reaktionsmischung 2,8 Liter Wasser zugesetzt und auf 25°C abgekühlt. Das Produkt wird abfiltriert, mit 2 Liter Wasser gewaschen und im Trockenschrank unter Inertisierung bei 70°C getrocknet.
Ausbeute: 257,5 g (91 % d. Th.)

### c. 1-[(3-Cyano-pyridin-2-yl)methyl]-3-methyl-7-(2-butin-1-yl)-8-(3-(R)-phthalimido-piperidin-1-yl)-xanthin

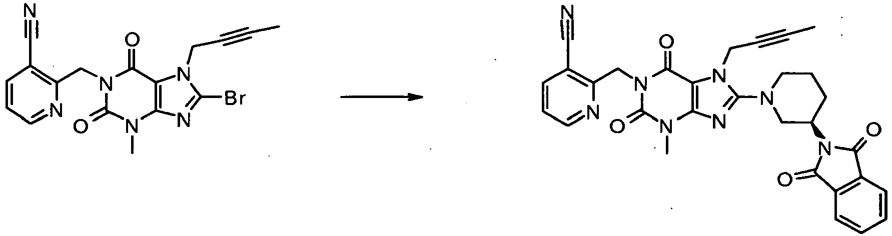

230 g (0,557 mol) 1-[(3-Cyano-pyridin-2-yl)methyl]-3-methyl-7-(2-butin-1-yl)-8-brom-xanthin, 318 g (0,835 mol) 3-(Phthalimido)piperidin D-Tartrat und 1,15 Liter N-Methyl-2-pyrrolidon werden im Reaktor vorgelegt. Der Reaktorinhalt wird auf 140°C erhitzt.

Nach Erreichen der Temperatur werden innerhalb von 20 Minuten 478 ml (2,78 mol) Diisopropylethylamin zudosiert und die Reaktionsmischung anschließend für 2 Stunden bei 140°C gerührt. Anschließend wird die Reaktionsmischung auf 75°C abgekühlt und mit 720 ml Methanol verdünnt. Danach werden bei 68-60°C 2,7 Liter Wasser zugegeben und auf 25°C abgekühlt. Das Produkt wird abfiltriert und mit 2 Liter Wasser gewaschen. Es wird im Trockenschrank unter Inertisierung bei 70°C getrocknet.

Das so erhaltene Rohprodukt wird anschließend in 1 Liter Methanol in der Siedehitze verrührt, heiß filtriert mit 200 ml Methanol gewaschen und anschließend bei 70°C unter Inertisierung getrocknet.
Ausbeute: 275 g (88 % d. Th.)

### d. 1-[(3-Cyano-pyridin-2-yl)methyl]-3-methyl-7-(2-butin-1-yl)-8-(3-(R)-amino-piperidin-1-yl)-xanthin

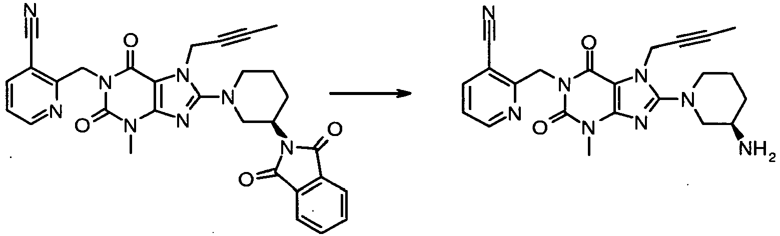

412,5 g (0,733 mol) 1-[(3-Cyano-pyridin-2-yl)methyl]-3-methyl-7-(2-butin-1-yl)-8-(3-(R)-phthalimido-piperidin-1-yl)-xanthin werden in 4125 ml Toluol auf 80°C erhitzt. Anschließend werden bei 75-80°C 445 ml Ethanolamin (7,33 mol) zur Suspension zugeben. Zur Vervollständigung der Reaktion wird 2 Stunden bei 80-85°C nachgerührt, wobei die Feststoffe in Lösung gehen. Anschließend werden die Phasen getrennt. Die Ethanolamin-Phase wird zweimal mit warmen Toluol (je 1 Liter) extrahiert. Die vereinigten Toluol-Phasen werden zweimal mit je 2 Liter 75-80°C warmen Wasser gewaschen. Die Toluol-Phasen werden mit Natriumsulfat getrocknet, filtriert und anschließend durch Destillation im Vakuum auf ein Volumen von ca. 430 ml reduziert. Anschließend wird bei 50-55°C 1 Liter tert.-Butylmethylether zudosiert und danach auf 0-5°C abgekühlt. Das Produkt wird durch Filtration isoliert, mit tert.-Butylmethylether nachgewaschen und im Trockenschrank bei 60°C getrocknet.
Ausbeute: 273,25 g (86,2% d.Th.)
Schmelzpunkt: 188 ± 3 °C (wasserfreie Form)

Die wasserfreie Form von 1-[(3-Cyano-pyridin-2-yl)methyl]-3-methyl-7-(2-butin-1-yl)-8-(3-(R)-amino-piperidin-1-yl)-xanthin ist bei relativen Luftfeuchten bis ca. 50 % stabil, oberhalb 50 % r.F. nimmt diese Form ca. 4 % Wasser auf und wandelt sich dabei in ein Monohydrat um, wie aus dem Sorptionsdiagramm in Figur 4 leicht zu erkennen ist. Wird die relative Feuchte anschließend wieder auf 50 % oder kleiner gebracht, bildet sich die wasserfreie Form wieder zurück, d.h. die Umwandlung in das Monohydrat ist voll reversibel.

**Tabelle 1: Röntgenreflexe mit Intensitäten (normiert) für die wasserfreie Form von 1-[(3-Cyano-pyridin-2-yl)methyl]-3-methyl-7-(2-butin-1-yl)-8-(3-(R)-amino-piperidin-1-yl)-xanthin**

| **2Θ** | **dₕₖₗ** | **Intensität** |
|---|---|---|
| **[°]** | **[Å]** | **I/Iₒ [%]** |
| 8,17 | 10,81 | 22 |
| 8,37 | 10,56 | 19 |
| 10,29 | 8,59 | 100 |
| 11,40 | 7,76 | 14 |
| 11,70 | 7,56 | 6 |
| 12,48 | 7,09 | 9 |
| 14,86 | 5,96 | 1 |
| 15,49 | 5,72 | 2 |
| 16,26 | 5,45 | 7 |
| 16,75 | 5,29 | 18 |
| 18,38 | 4,82 | 11 |
| 18,60 | 4,77 | 7 |
| 18,92 | 4,69 | 3 |
| 19,35 | 4,58 | 3 |
| 19,55 | 4,54 | 2 |
| 20,73 | 4,28 | 24 |
| 21,34 | 4,16 | 1 |
| 21,77 | 4,08 | 4 |
| 22,10 | 4,02 | 7 |
| 22,60 | 3,93 | 4 |
| 22,86 | 3,89 | 5 |
| 23,09 | 3,85 | 5 |
| 23,47 | 3,79 | 11 |
| 24,30 | 3,66 | 12 |
| 24,66 | 3,61 | 5 |
| 25,58 | 3,48 | 2 |
| 26,02 | 3,42 | 2 |
| 26,52 | 3,36 | 13 |
| 27,15 | 3,28 | 3 |
| 27,60 | 3,23 | 2 |
| 28,22 | 3,16 | 4 |
| 28,60 | 3,12 | 2 |
| 28,84 | 3,09 | 3 |
| 29,80 | 3,00 | 5 |
| 30,02 | 2,97 | 8 |

**Tabelle 2: Röntgenreflexe mit Intenistäten (normiert) für das Monohydrat von 1-[(3-Cyano-pyridin-2-yl)methyl]-3-methyl-7-(2-butin-1-yl)-8-(3-(R)-amino-piperidin-1-yl)-xanthin**

| **2 Θ** | **dₕₖₗ** | **Intensität** |
|---|---|---|
| **[°]** | **[Å]** | **I/Iₒ [%]** |
| 8,00 | 11,05 | 12 |
| 8,45 | 10,46 | 26 |
| 9,27 | 9,54 | 5 |
| 10,43 | 8,48 | 100 |
| 11,45 | 7,72 | 23 |
| 11,74 | 7,53 | 4 |
| 12,53 | 7,06 | 6 |
| 15,91 | 5,57 | 8 |
| 16,01 | 5,53 | 7 |
| 16,73 | 5,30 | 29 |
| 16,94 | 5,23 | 17 |
| 17,99 | 4,93 | 11 |
| 18,43 | 4,81 | 15 |
| 18,95 | 4,68 | 7 |
| 19,31 | 4,59 | 2 |
| 20,54 | 4,32 | 36 |
| 20,85 | 4,26 | 30 |
| 21,86 | 4,06 | 19 |
| 22,13 | 4,01 | 8 |
| 22,70 | 3,91 | 7 |
| 22,96 | 3,87 | 15 |
| 23,43 | 3,79 | 8 |
| 24,56 | 3,62 | 18 |
| 25,45 | 3,50 | 2 |
| 25,84 | 3,44 | 14 |
| 25,99 | 3,43 | 14 |
| 26,58 | 3,35 | 6 |
| 26,88 | 3,31 | 4 |
| 28,06 | 3,18 | 3 |
| 29,04 | 3,07 | 4 |
| 29,29 | 3,05 | 9 |
| 30,22 | 2,96 | 6 |

### Beispiel 3

### 1-[(3-Cyano-pyridin-2-yl)methyl]-3-methyl-7-(2-butin-1-yl)-8-[(R)-3-amino-piperidin-1-yl]-xanthin-Monohydrochlorid

5,00 g 1-[(3-Cyano-pyridin-2-yl)methyl]-3-methyl-7-(2-butin-1-yl)-8-[(*R*)-3-amino-piperidin-1-yl]-xanthin-Base werden in 50 ml Methanol gelöst. Anschließend werden 3,0 ml einer 3,9 molaren Lösung von Chlorwasserstoff in Isopropanol zugegeben. Das Lösemittel wird abdestilliert und der Rückstand in 40 ml Ethylacetat suspendiert und zum Rückfluß erhitzt, wobei sich ein Niederschlag bildet. Es wird auf Raumtemperatur abgekühlt, der Niederschlag abfiltriert und mit wenig Ethylacetat gewaschen und getrocknet.
Das Produkt wird anschließend aus absolutem Ethanol umkristallisiert.
Ausbeute: 2,7 g (50 % d.Th.)
Schmelzpunkt: 265 ± 5 °C (unter Zersetzung)

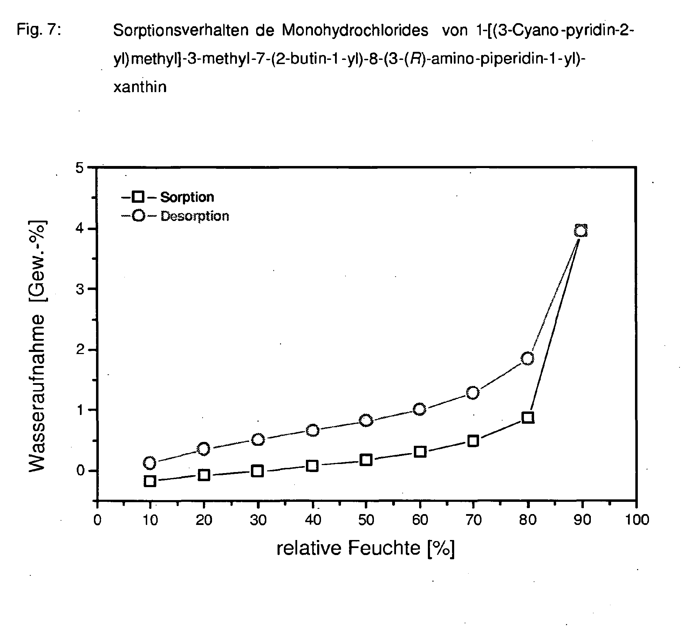

Das Monohydrochlorid zeigt ein weniger stark ausgeprägtes hygroskopisches Verhalten, eine reversible Umwandlung in eine Hydratphase, wie es bei der freien Base zwischen 50 und 60 % r.F. beobachtet wird, ist nicht zu erkennen (siehe Sorptionsverhalten des Monohydrochlorides in Figur 7). Erst bei sehr hohen relativen Luftfeuchten (> 80 % r.F.) nimmt auch das Monohydrochlorid Wasser auf. Luftfeuchteabhängige Röntgenpulveraufnahmen zeigen, dass keine Phasenumwandlung oberhalb 80 % r.F. für das Monohydrochlorid stattfindet.

**Tabelle 3: Röntgenreflexe mit Intenistäten (normiert) für die wasserfreie Form von 1-[(3-Cyano-pyridin-2-yl)methyl]-3-methyl-7-(2-butin-1-yl)-8-[(R)-3-amino-piperidin-1-yl]-xanthin-Monohydrochlorid**

| **2 Θ** | **dₕₖₗ** | **Intensity** |
|---|---|---|
| **[°]** | **[Å]** | **I/Iₒ [%]** |
| 17,95 | 4,92 | 5 |
| 10,38 | 8,51 | 100 |
| 8,99 | 9,83 | 22 |
| 6,80 | 13,01 | 26 |
| 5,97 | 14,82 | 1 |
| 5,19 | 17,07 | 12 |
| 4,99 | 17,78 | 5 |
| 4,74 | 18,72 | 8 |
| 4,44 | 20,00 | 10 |
| 4,31 | 20,60 | 13 |
| 3,98 | 22,33 | 10 |
| 3,93 | 22,61 | 11 |
| 3,80 | 23,38 | 4 |
| 3,57 | 24,91 | 11 |
| 3,50 | 25,43 | 11 |
| 3,32 | 26,87 | 4 |
| 3,15 | 28,35 | 2 |
| 2,96 | 30,20 | 1 |
| 2,89 | 30,97 | 2 |
| 2,80 | 31,92 | 5 |
| 2,74 | 32,63 | 2 |
| 2,57 | 34,89 | 2 |

### Beispiel 4

### 1-[(3-Cyano-pyridin-2-yl)methyl]-3-methyl-7-(2-butin-1-yl)-8-[(R)-3-amino-piperidin-1-yl]-xanthin-Dihydrochlorid

1-[(3-Cyano-pyridin-2-yl)methyl]-3-methyl-7-(2-butin-1-yl)-8-[(*R*)-3-amino-piperidin-1-yl]-xanthin-Base (1,00 g; 2,31 mmol) werden in 9,5 ml abs. Ethanol und 0,5 ml Methyl-tert.-butylether bei Siedetemperatur gelöst. Anschließend werden 1,2 ml einer 3,9 molaren Lösung von Chlorwasserstoff in Isopropanol zugegeben. Es bildet sich ein Niederschlag. Nach Abkühlen auf Raumtemperatur wird filtriert, mit wenig MTBE nachgewaschen und getrocknet.
Ausbeute: 1,04 g (89,0% d.Th.)
Schmelzpunkt: 205 ± 5 °C (unter Zersetzung); ab ca. 150 °C Abgabe von gasförmigem HCl

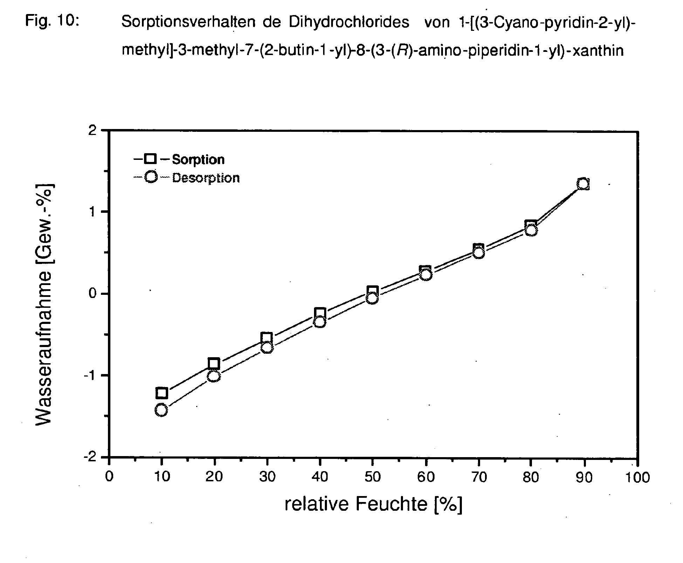

Das Dihydrochlorid zeigt ebenfalls ein unauffälliges hygroskopisches Verhalten, eine reversible Umwandlung in eine Hydratphäse, wie es bei der freien Base zwischen 50 und 60 % r.F. beobachtet wird, ist nicht zu erkennen (siehe Sorptionsverhalten des Dihydrochlorides in Figur 10). Das Dihydrochlorid nimmt kontinuierlich über den gesamten Luftfeuchtebereich etwas Wasser auf. Luftfeuchteabhängige Röntgenpulveraufnahmen zeigen, dass ebenfalls keine Phasenumwandlung im Luftfeuchtebereich 10- 90 r.F. stattfindet.

**Tabelle 4: Röntgenreflexe mit Intenistäten (normiert) für die wasserfreie Form von 1-[(3-Cyano-pyridin-2-yl)methyl]-3-methyl-7-(2-butin-1-yl)-8-[(R)-3-amino-piperidin-1-yl]-xanthin-Dihydrochlorid**

| **2Θ** | **dₕₖₗ** | **Intensität** |
|---|---|---|
| **[°]** | **[Å]** | **I/Iₒ [%]** |
| 7,20 | 12,26 | 3 |
| 7,86 | 11,24 | 27 |
| 9,34 | 9,46 | 3 |
| 10,04 | 8,81 | 54 |
| 11,53 | 7,67 | 100 |
| 11,87 | 7,45 | 5 |
| 12,20 | 7,25 | 19 |
| 13,25 | 6,68 | 2 |
| 13,97 | 6,33 | 5 |
| 14,47 | 6,12 | 15 |
| 15,77 | 5,61 | 18 |
| 16,65 | 5,32 | 4 |
| 17,15 | 5,17 | 2 |
| 18,05 | 4,91 | 11 |
| 18,50 | 4,79 | 37 |
| 18,80 | 4,72 | 14 |
| 20,16 | 4,40 | 15 |
| 20,46 | 4,34 | 2 |
| 21,03 | 4,22 | 3 |
| 21,36 | 4,16 | 10 |
| 21,64 | 4,10 | 18 |
| 22,54 | 3,94 | 9 |
| 23,17 | 3,84 | 13 |
| 23,90 | 3,72 | 15 |
| 24,57 | 3,62 | 7 |
| 24,86 | 3,58 | 9 |
| 25,06 | 3,55 | 11 |
| 25,39 | 3,50 | 13 |
| 25,95 | 3,43 | 6 |
| 26,14 | 3,41 | 13 |
| 26,52 | 3,36 | 12 |
| 26,79 | 3,32 | 43 |
| 27,24 | 3,27 | 4 |
| 27,66 | 3,22 | 3 |
| 28,15 | 3,17 | 6 |
| 29,22 | 3,05 | 3 |
| 29,48 | 3,03 | 23 |
| 30,16 | 2,96 | 18 |

Die Schmelzpunkte wurden mittels DSC ermittelt, es wurde hierfür ein Gerät der Fa. Mettler-Toledo (Typ: DSC 821) eingesetzt. Als Schmelztemperatur wurde die Onset-Temperatur des entsprechenden Schmelzpeaks im DSC-Diagramm verwendet. Es wurden Heizraten von 10 K/min verwendet, die Experimente wurden unter Stickstoffatmosphäre durchgeführt.

Die Röntgenpulverdiagramme wurden bis auf eine Ausnahme mit einem STOE Stadi P Röntgepulverdiffraktometer aufgenommen. Dieses Diffraktometer arbeitet mit CuK_{α1} - Strahlung (λ = 1.5406 Å) und einem ortsempfi ndlichen Detektor. Der Röntgengenerator wurde mit 40 mA und 40 kV betrieben.

Das Röntgenpulverdiagramm des Monohydrates der freien Base wurde mit einem Bruker D8 Advance Röntgenpulverdiffraktometer aufgenommen, auf das eine spezielle Luftfeuchtezelle von der Fa. MRI aufgesetzt wurde. Das Diagramm wurde bei ca. 72 % r.F. aufgenommen. Das Bruker D8 Advance arbeitet mit CuK_{α} - Strahlung (λ = 1.5418 Å) und ebenfalls einem ortsempfindlichen Detektor. Der Röntgengenerator wurde mit 30 mA und 40 kV betrieben.

### Beispiel 5

### Dragées mit 75 mg Wirksubstanz

### 1 Dragéekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.
Dragéegewicht: 245 mg.

### Beispiel 6

### Tabletten mit 100 mg Wirksubstanz

Zusammensetzung:

### 1 Tablette enthält:

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 7

### Tabletten mit 150 mg Wirksubstanz

### Zusammensetzung:

### 1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1.0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden

### Tabletten gepreßt.

| | |
|---|---|
| Tablettengewicht: | 300 mg |
| Stempel: | 10 mm, flach |

### Beispiel 8

### Hartoelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 9

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 10

### Suspension mit 50 mg Wirksubstanz

### 100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 11

### Ampullen mit 10 mg Wirksubstanz

### Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 12

### Ampullen mit 50 mg Wirksubstanz

### Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Ein Hydrochlorid Salz von 1-[(3-Cyano-pyridin-2-yl)methyl]-3-methyl-7-(2-buun-1-yl)-8-(3-amino-piperidin-1-yl)-xanthin.

2. Ein Monohydrochloridsalz oder Dihydrochloridsalz von 1-[(3-Cyano-pyridin-2-yl)methyl]-3-methyt-7-(2-butin-1-yl)-8-[(*R*)-3-amlno-piperidin-1-yl]-xanthin.

3. Ein Monohydrochlorid- oder Dihydrochloridsalz von 1-[(3-Cyano-pyridin-2-yl)methyl]-3-methyl-7-(2-butin-1-yl)-8-[(R)-(3-amino-piperidin-1-yl]-xanthin in kristalliner Nicht-Hydrat-Form.

4. 1-[(3-Cyario-pyridin-2-yl)methyl]-3-methyl-7-(2-butin-1-yl)-8-[(*R*)-3-amino-piperidin-1-yl]-xanthin - Monohydrochlorid.

5. 1-[(3-Cyano-pyridin-2-yl)methyl]-3-methyl-7-(2-butin-1-yl)-8-[(*R*)-3-amino-piperidin-1-yl]-xanthin - Dihydrochlorid.

6. Das Monohydrochloridsalz von 1-[(3-Cyano-pyridin-2-yl)methyl]-3-methyl-7-(2-butin-1-yl)-8-[(*R*)-3-amino-piperidin-1-yl]-xanthin, das einen Schmelzpunkt von 265 ± 5 °C aufweist.

7. Das Dihydrochloridsalz von 1-[(3-Cyano-pyridin-2-yl)methyl]-3-methyl-7-(2-butin-1-yl)-8-[(*R*)-3-amino-piperidin-1-yl]-xanthin, das einen Schmelzpunkt von 205 ± 5 °C aufweist.

8. Das Monohydrochioridsalz von 1-[(3-Cyano-pyridin-2-yl)methyl]-3-methyl-7-(2-butin-1-yl)-8-[(*R*)-3-amino-piperidin-1-yl]-xanthin, wobei das Röntgenpulverdiagramm dieses Monohydrochlorids charakteristische d Werte bei 10.38 Å, 8.99 Å, 6.80 Å, 5.19 Å, 4.44 Å, 4.31 Å, 3.98 Å, 3.93 Å, 3.57 Å, 3.50 Å umfasst.

9. Das Dihydrochloridsalz von 1-[(3-Cyano-pyridin-2-yl)methyl]-3-methyl-7-(2-butin-1-yl)-8-[(*R*)-3-amino-piperidin-1-yl]-xanthin, wobei das Röntgenpulverdiagramm dieses Dihydrochlorids charakteristische d Werte bei 11.24 Å, 8.81 Å, 7.67 Å, 7.25 Å, 5.61 Å, 4.79 Å, 4.10 Å, 3.32 Å, 3.03 Å, 2.96 Å umfasst.

10. Verwendung einer Verbindung gemäß einem der Ansprüche 1 bis 9 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

11. Verwendung einer Verbindung gemäß einem der Ansprüche 1 bis 9 zur Herstellung eines Arzneimittels, das zur Behandlung von Diabetes mellitus Typ II oder Adipositas geeignet ist.

12. Pharmazeutische Zusammensetzung enthaltend ein Salz gemäß einem der Ansprüche 1 bis 9 neben gegebenenfalls einem oder mehreren inerten Trägerstoffen und/oder Verdünnungsmitteln.

13. Pharmazeutische Zusammensetzung enthaltend das Monohydrochloridsalz von 1-[(3-Cyano-pyndin-2-yl)methyl]-3-methyl-7-(2-butin-1-yl)-8-[(*R*)-3-amino-piperidin-1-yl]-xanthin neben gegebenenfalls einem oder mehreren inerten Trägerstoffen und/oder Verdünnungsmitteln.

14. Pharmazeutische Zusammensetzung enthaltend das Dihydrochloridsalz von 1-[(3-Cyano-pyridin-2-yl)methyl]-3-methyml-7-(2-butin-1-yl)-8-[(*R*)-3-amino-piperidin-1-yl]-xanthin neben gegebenenfalls einem oder mehreren inerten Trägerstoffen und/oder Verdünnungsmitteln.

15. Pharmazeutische Zusammensetzung enthaltend das Monohydrochloridsalz von 1-[(3-Cyano-pyridin-2-yl)methyl]-3-methyl-7-(2-butin-1-yl)-8-[(*R*)-3-amino-piperidin-1-yl]-xanthin gemäß Anspruch 4, 6 oder 8 neben gegebenenfalls einem oder mehreren inerten Trägerstoffen und/oder Verdünnungsmitteln.

## Claims

1. A hydrochloride salt of 1-[(3-cyano-pyridin-2-yl)methyl]-3-methyl-7-(2-butyn-1-yl)-8-(3-amino-piperidin-1-yl)-xanthine.

2. A monohydrochloride salt or dihydrochloride salt of 1-[(3-cyano-pyridin-2-yl)methyl]-3-methyl-7-(2-butyn-1-yl)-8-[(*R*)-3-amino-piperidin-1-yl]-xanthine.

3. A monohydrochloride or dihydrochloride salt of 1-[(3-cyano-pyridin-2-yl)methyl]-3-methyl-7-(2-butyn-1-yl)-8-[(*R*)-3-amino-piperidin-1-yl]-xanthine in crystalline non-hydrate form.

4. 1-[(3-Cyano-pyridin-2-yl)methyl]-3-methyl-7-(2-butyn-1-yl)-8-[(*R*)-3-amino-piperidin-1-yl]-xanthine monohydrochloride.

5. 1-[(3-Cyano-pyridin-2-yl)methyl]-3-methyl-7-(2-butyn-1-yl)-8-[(*R*)-3-amino-piperidin-1-yl]-xanthine dihydrochloride.

6. The monohydrochloride salt of 1-[(3-cyano-pyridin-2-yl)methyl]-3-methyl-7-(2-butyn-1-yl)-8-[(*R*)-3-amino-piperidin-1-yl]-xanthine, which has a melting point of 265 ± 5°C.

7. The dihydrochloride salt of 1-[(3-cyano-pyridin-2-yl)methyl]-3-methyl-7-(2-butyn-1-yl)-8-[(*R*)-3-amino-piperidin-1-yl]-xanthine, which has a melting point of 205 ± 5°C.

8. The monohydrochloride salt of 1-[(3-cyano-pyridin-2-yl)methyl]-3-methyl-7-(2-butyn-1-yl)-8-[(*R*)-3-amino-piperidin-1-yl]-xanthine, wherein the X-ray powder diagram of this monohydrochloride includes characteristic d values at 10.38 Å, 8.99 Å, 6.80 Å, 5.19 Å, 4.44 Å, 4.31 Å, 3.98 Å, 3.93 Å, 3.57 Å, 3.50 Å.

9. The dihydrochloride salt of 1-[(3-cyano-pyridin-2-yl)methyl]-3-methyl-7-(2-butyn-1-yl)-8-[(*R*)-3-amino-piperidin-1-yl]-xanthine, wherein the X-ray powder diagram of this dihydrochloride includes characteristic d values at 11.24 Å, 8.81 Å, 7.67 Å, 7.25 Å, 5.61 Å, 4.79 Å, 4.10 Å, 3.32 Å, 3.03 Å, 2.96 Å.

10. Use of a compound according to one of claims 1 to 9 for preparing a medicament that is suitable for the treatment of type I and type II diabetes mellitus, arthritis, obesity, allograft transplantation and osteoporosis caused by calcitonin.

11. Use of a compound according to one of claims 1 to 9 for preparing a medicament that is suitable for the treatment of type II diabetes mellitus or obesity.

12. Pharmaceutical composition containing a salt according to one of claims 1 to 9 optionally together with one or more inert carriers and/or diluents.

13. Pharmaceutical composition containing the monohydrochloride salt of 1-[(3-cyano-pyridin-2-yl)methyl]-3-methyl-7-(2-butyn-1-yl)-8-[(*R*)-3-amino-piperidin-1-yl]-xanthine, optionally together with one or more inert carriers and/or diluents.

14. Pharmaceutical composition containing the dihydrochloride salt of 1-[(3-cyano-pyridin-2-yl)methyl]-3-methyl-7-(2-butyn-1-yl)-8-[(*R*)-3-amino-piperidin-1-yl]-xanthine, optionally together with one or more inert carriers and/or diluents.

15. Pharmaceutical composition containing the monohydrochloride salt of 1-[(3-cyano-pyridin-2-yl)methyl]-3-methyl-7-(2-butyn-1-yl)-8-[(*R*)-3-amino-piperidin-1-yl]-xanthine according to claim 4, 6 or 8, optionally together with one or more inert carriers and/or diluents.

## Revendications

1. Sel chlorhydrate de 1-[(3-cyanopyridin-2-yl)-méthyl]-3-méthyl-7-(2-butin-l-yl)-8-(3-amino-pipéridin-1-yl)-xanthine.

2. Sel monochlorhydrate ou sel dichlorhydrate de 1-[(3-cyanopyridin-2-yl)méthyl]-3-méthyl-7-(2-butin-1-yl)-8-[(R)-3-aminopipéridin-1-yl]-xanthine.

3. Sel monochlorhydrate ou dichlorhydrate de 1-[(3-cyanopyridin-2-yl)méthyl]-3-méthyl-7-(2-butin-1-yl)-8-[(R)-3-aminopipéridin-1-yl]-xanthine sous forme cristalline non hydratée.

4. Monochlorhydrate de 1-[(3-cyanopyridin-2-yl)méthyl]-3-méthyl-7-(2-butin-1-yl)-8-[(R)-3-aminopipéridin-1-yl]-xanthine.

5. Dichlorhydrate de 1-[(3-cyanopyridin-2-yl)-méthyl]-3-méthyl-7-(2-butin-1-yl)-8-[(R)-3-aminopipéridin-1-yl]-xanthine.

6. Sel monochlorhydrate de 1-[(3-cyanopyridin-2-yl)méthyl]-3-méthyl-7-(2-butin-1-yl)-8-[(R)-3-aminopipéridin-1-yl]-xanthine qui présente un point de fusion de 265 ± 5° C.

7. Sel dichlorhydrate de 1-[(3-cyanopyridin-2-yl)méthyl]-3-méthyl-7-(2-butin-1-yl)-8-[(R)-3-aminoplpéridin-1-yl]-xanthine qui présente un point de fusion de 205 ± 5° C.

8. Sel monochlorhydrate de 1-[(3-cyanopyridin-2-yl)méthyl]-3-méthyl-7-(2-butin-1-yl)-8-[(R)-3-aminopipéridin-1-yl]-xanthine, dans lequel le diagramme sur poudre radiographique de ce monochlorhydrate comprend des valeurs d caractéristiques à 10,38 Å, 8,99 Å, 6,80 Å, 5,19 Å, 4,44 Å, 4,31 Å, 3,98 Å, 3,93 Å, 3,57 Å, 3,50 Å.

9. Sel dichlorhydrate de 1-[(3-cyanopyridin-2-yl)méthyl]-3-méthyl-7-(2-butin-1-yl)-8-[(R)-3-aminopipéridin-1-yl]-xanthine, dans lequel le diagramme sur poudre radiographique de ce dichlorhydrate comprend des valeurs d caractéristiques à 11,24 Å, 8,81 Å, 7,67 Å, 7,25 Å, 5,61 Å, 4,79 Å, 4,10 Å, 3,32 Å, 3,03 Å, 2,96 Å.

10. Utilisation d'un composé selon l'une des revendications 1 à 9, pour la production d'un médicament qui est approprié pour le traitement du diabète sucré de type I et de type II, de l'arthrite, de l'adiposité, des allogreffes et de l'ostéoporose induite par la calcitonine.

11. Utilisation d'un composé selon l'une des revendications 1 à 9, pour la production d'un médicament qui est approprié pour le traitement du diabète sucré de type II ou de l'adiposité.

12. Composition pharmaceutique contenant un sel selon l'une des revendications 1 à 9, parallèlement à éventuellement un ou plusieurs adjuvants et/ou diluants inertes.

13. Composition pharmaceutique contenant le Sel monochlorhydrate de 1-[(3-cyanopyridin-2-yl)méthyl]-3-méthyl-7-(2-butin-1-yl)-8-[(R)-3-aminopipéridin-1-yl]-xanthine avec éventuellement un ou plusieurs adjuvants et/ou diluants inertes.

14. Composition pharmaceutique contenant le Sel dichlorhydrate de 1-[(3-cyanopyridin-2-yl)-méthyl]-3-méthyl-7-(2-butin-1-yl)-8-[(R)-3-aminopipéridin-1-yl]-xanthine avec éventuellement un ou plusieurs adjuvants et/ou diluants inertes.

15. Composition pharmaceutique contenant le Sel monochlorhydrate de 1-[(3-cyanopyridin-2-yl)méthyl]-3-méthyl-7-(2-butin-1-yl)-8-[(R)-3-aminopipéridin-1-yl]-xanthine selon la revendication 4, 6 ou 8, avec éventuellement un ou plusieurs adjuvants et/ou diluants inertes.
